(19) Europäisches Patentamt

European Patent Office

Office européen des brevets

(11) **EP 1 318 136 B1**

(12) **EUROPÄISCHE PATENTSCHRIFT**

(45) Veröffentlichungstag und Bekanntmachung des Hinweises auf die Patenterteilung:
**23.02.2005 Patentblatt 2005/08**

(51) Int Cl.7: **C07C 51/245**, C07C 53/02, C07C 53/08, C07C 53/122, C07C 53/124

(21) Anmeldenummer: **02026493.3**

(22) Anmeldetag: **28.11.2002**

(54) **Verfahren zur Herstellung von gesättigten Carbonsäuren mit ein bis vier C-Atomen durch Gasphasenoxidation von 2-butanon**

Process for the production of saturated carboxylic acids with 1 to 4 carbon atoms via gas phase oxidation of 2-butanone

Procédé de préparation d'acides saturés contenant 1 à 4 atomes carbone par oxidation en phase gaseuze de 2-butanone

(84) Benannte Vertragsstaaten:
**AT BE BG CH CY CZ DE DK EE ES FI FR GB GR IE IT LI LU MC NL PT SE SK TR**

(30) Priorität: **06.12.2001 DE 10159834**

(43) Veröffentlichungstag der Anmeldung:
**11.06.2003 Patentblatt 2003/24**

(73) Patentinhaber: **Consortium für elektrochemische Industrie GmbH**
**81379 München (DE)**

(72) Erfinder:
• **Rüdinger, Christoph, Dr.**
**82319 Starnberg (DE)**
• **Voit, Harald Herbert, Dr.**
**84571 Reischach (DE)**
• **Eberle, Hans-Jürgen**
**81477 München (DE)**

(74) Vertreter: **Schuderer, Michael, Dr. et al**
**Wacker-Chemie GmbH**
**Zentralabteilung Patente**
**Marken und Lizenzen**
**Hanns-Seidel-Platz 4**
**81737 München (DE)**

(56) Entgegenhaltungen:
WO-A-98/23371          DE-B- 2 525 296
US-A- 3 627 823

**Beschreibung**

Die Erfindung betrifft ein Verfahren zur Herstellung von gesättigten Carbonsäuren mit ein bis vier C-Atomen, insbesondere von Essigsäure durch Gasphasenoxidation von 2-Butanon unter Verwendung eines Schalenkatalysators.

Es ist bekannt, daß gesättigten Carbonsäuren mit ein bis vier C-Atomen und insbesondere Essigsäure durch Gasphasenoxidation von kurzkettigen aliphatischen Verbindungen, besonders Butenen und daraus erhältlichen alkoholischen oder ketonischen Oxidationsprodukten mittels eines Katalysators hergestellt werden können. Bisher konnte jedoch noch kein wirtschaftlich und betriebstechnisch voll befriedigendes Verfahren gefunden werden.

Die DE-1 643 822 C3 beschreibt ein Verfahren zur Herstellung von Essigsäure durch katalytische Gasphasenoxidation von kurzkettigen aliphatischen Verbindungen mit Sauerstoff unter Verwendung von Aluminium- und Titanvanadatkatalysatoren. Diese Katalysatoren werden durch Ausfällen der Mischoxide aus den entsprechenden Lösungen hergestellt und als gekörnter Vollkontakt eingesetzt. Nachteilig bei diesen erfindungsgemäßen Katalysatoren ist der hohe Grad an Totaloxidation und die schlechte Beherrschbarkeit der Wärmeabfuhr, so daß bei der Oxidation von Methylethylketon (2-Butanon) nur eine Ausbeute von 75 % und eine maximale Essigsäureerzeugung von 64 g pro Liter Katalysator und Stunde erreicht werden. Darüber hinaus wird nur eine ungefähr 12 Gew.-%ige Rohsäure erhalten, die eine sehr aufwendige Aufkonzentrierung und Reinigung bedingt, um eine handelsübliche >99%ige reine Essigsäure zu erhalten.

Die DE-1 643 824 C beschreibt ein Verfahren zur Herstellung von konzentrierter Essigsäure durch katalytische Gasphasenoxidation von Butenen und daraus erhältlichen alkoholischen oder ketonischen Oxidationsprodukten unter Verwendung von Aluminium-, Antimon, Zinn- und Titanvanadatkatalysatoren in einer Folge von hintereinandergeschalteten Einzelreaktoren. Diese Katalysatoren werden durch Ausfällen der Mischoxide aus den entsprechenden Lösungen hergestellt und als gekörnter Vollkontakt eingesetzt. Nachteilig bei diesem erfindungsgemäßen Verfahren und den erfindungsgemäßen Katalysatoren ist der hohe Grad an Totaloxidation und die schlechte Beherrschbarkeit der Wärmeabfuhr, so daß bei der Oxidation von Methylethylketon (2-Butanon) nur eine Ausbeute von 66 % und eine maximale Essigsäureerzeugung von 126 g pro Liter Katalysator und Stunde erreicht werden. Darüber hinaus wird nur eine maximal 34 Gew.-%ige Rohsäure erhalten, die neben der schon sehr komplexen Hintereinanderschaltung von vier Reaktoren mit eigener Eduktdosierung und zwei Kondensatoren eine sehr aufwendige Aufkonzentrierung und Reinigung der Rohsäure bedingt um eine handelsübliche >99%ige reine Essigsäure zu erhalten.

Die DE-1 643 824 C beschreibt ferner, daß die Verwendung von 2-Butanon (Methylethylketon) als Edukt gegenüber der Verwendung von n-Butenen, wie 1-Buten, trans-2-Buten, cis-2-Buten schlechtere Ergebnisse in Bezug auf die Höhe der Essigsäureausbeute sowie der erzielbaren Raum-Zeit-Leistung und Rohsäurekonzentration liefert.

Die DE-1279011 B beschreibt ein Verfahren zur Herstellung von Essigsäure durch katalytische Gasphasenoxidation von Buten mit Sauerstoff unter Verwendung von Aluminium- und Titanvanadatkatalysatoren. Diese Katalysatoren werden ebenfalls durch Ausfällen der Mischoxide aus den entsprechenden Lösungen hergestellt, wobei die Mischoxide gegebenenfalls noch mit inerten Materialien wie Kieselsäure vermischt werden können. Der Katalysator wird als feinteiliges Pulver in Wirbelbettreaktoren eingesetzt.

Nachteilig bei derartigen Vollkontakt-Katalysatoren ist der hohe Grad an Totaloxidation.

Zur Verbesserung der Ausbeute derartiger Katalysatoren wird in der DE-2016681 A vorgeschlagen, die Katalysatoren vor der Calcinierung mit einem Oxidationsmittel vorzubehandeln. In der DE-A 2354425 C3 (US 3,954,857) wird zur Verbesserung der Selektivität die Behandlung des calcinierten Titan-Vanadium-Mischkatalysators mit Salzsäure vorgeschlagen. Die Katalysatoren werden als Vollkontakt, gegebenenfalls im Gemisch mit inerten Trägermaterialien wie Kieselsäure, eingesetzt.

Ein weiterer aus dem Stand der Technik bekannter Ansatzpunkt, zur Verbesserung der Aktivität von Titan-Vanadium-Mischkatalysatoren bei der Gasphasenoxidation von Butenen zu Essigsäure, ist der Einsatz von Titandioxid in definierter Kristallform oder mit definierter Oberfläche. In der DE-A 2026744 (US-A 3917682) werden Titan-Vanadium-Mischkatalysatoren beschrieben, deren Titandioxid-Komponente überwiegend als Rutil vorliegt. Die Katalysatoren können in Pulverform oder zu Formkörpern gepreßt eingesetzt werden. Aus der US-A 4448897 sind Ti-Vanadium-Katalysatoren zur Butenoxidation bekannt, welche Titandioxid mit einer BET-Oberfläche größer 40 m$^2$/g enthalten. Die Katalysatoren werden ebenfalls in Pulverform oder als Preßling eingesetzt.

Aus dem Stand der Technik ist weiter bekannt, die Selektivität von Titan-Vanadium-Katalysatoren bei der Butenoxidation dadurch zu verbessern, daß man den Titandioxid-Anteil ganz oder teilweise durch andere Metalloxide substituiert. Die DE 2110876 A (GB 1333306) beispielsweise beschreibt Katalysatoren, welche Oxide von Molybdän, Zinn und Vanadium als Aktivkomponenten enthalten. Die Katalysatoren werden in Pulverform eingesetzt, wobei der Mischoxid-Katalysator gegebenenfalls auch auf feinteilige Trägermaterialien wie Siliciumdioxid aufgetragen werden kann. Aus der US 4,146,734 ist bekannt, Vanadium-Mischoxide einzusetzen, welche mit Cer und weiteren Übergangsmetalloxiden dotiert sind. Der Katalysator wird als feinteilges Granulat eingesetzt, kann aber auch als Präzipitat auf feinteilige, inerte Träger aufgetragen werden.

Aus der DE 2235103 C3 sind Titan-Vanadium-Mischoxid-Katalysatoren zur Gasphasenoxidation von Butenen in Form von Trägerkatalysatoren bekannt, bei denen ein vorgeformter poröser Träger mit der Mischlösung der Katalysatorkomponenten getränkt wird.

Allen diesen Katalysatoren ist gemeinsam, daß es sich um Vollkontakt-Katalysatoren handelt, bei denen die Aktivkomponenten selbst als Pulver oder Preßlinge eingesetzt werden, oder mit feinteiligen Trägermaterialien verdünnt als Pulver oder Preßling eingesetzt werden. Als Vollkontakte sind auch mit Aktivkomponente durchtränkte poröse Träger gemäß der DE 2235103 C3 zu verstehen, da auch hier das gesamte Katalysatorvolumen katalytisch aktiv ist.

Nachteilig bei all diesen Verfahren und Katalysatoren ist der hohe Grad an Totaloxidation, die bei hohen Raum-Zeit-Leistungen schlechte Beherrschbarkeit der Oxidationsreaktion und die geringe Essigsäurekonzentration in der Rohsäure. Diese Nachteile führen zu hohen Produktionskosten der Essigsäure.

Die EP 0 951 351 und die EP 0 960 874 beschreiben einen Schalenkatalysator zur Herstellung von Essigsäure durch Gasphasenoxidation von Kohlenwasserstoffen mit vier Kohlenstoffatomen ($C_4$-Kohlenwasserstoffe).

Die EP 0 960 875 und die EP 1 035 101 beschreiben Verfahren zur Herstellung von gesättigten Carbonsäuren mit 1 bis 4 C-Atomen aus gesättigten und ungesättigten $C_4$-Kohlenwasserstoffen unter Verwendung von Schalenkatalysatoren.

Diese Verfahren haben den Nachteil, daß zwar einerseits hohe Essigsäureausbeuten erzielt werden, andererseits jedoch die volumenbezogene Essigsäureproduktiviät (Raum-Zeit-Leistung) und die Konzentration der Rohsäure nicht voll befriedigen. Folglich ist die Ausführung dieser Verfahren immer noch mit hohen Reaktor- und Aufarbeitungskosten verbunden.

Ferner zeigte sich, daß durch die Erhöhung des n-Butenanteils im Rohstoff oder die Steigerung der Reaktorbelastung die volumenspezifische Essigsäureproduktivität der bekannten Verfahren zur Produktion von Essigsäure aus $C_4$-Kohlenwasserstoff-Gemischen nur unwesentlich und/oder mit Selektivitätseinbußen gesteigert werden kann.

Es bestand daher die Aufgabe, ein Verfahren zur Herstellung von gesättigten Carbonsäuren mit ein bis vier C-Atomen, insbesondere von Essigsäure durch Gasphasenoxidation zur Verfügung zu stellen, welches die Nachteile des Stands der Technik vermeidet.

Es wurde überraschend gefunden, daß die Herstellung von gesättigten Carbonsäuren mit ein bis vier C-Atomen, insbesondere von Essigsäure durch Gasphasenoxidation besonders wirtschaftlich ist, wenn Schalenkatalysatoren verwendet werden, bei denen die aktive Masse als dünne Schicht auf einem unporösen Trägerkörper aufgebracht ist und ein 2-Butanon enthaltendes Edukt eingesetzt wird.

Gegenstand der Erfindung ist ein Verfahren zur Herstellung von gesättigten Carbonsäuren mit ein bis vier Kohlenstoffatomen durch Gasphasenoxidation, dadurch gekennzeichnet, daß 2-Butanon an einem Schalenkatalysator umgesetzt wird, enthaltend einen inerten unporösen Trägerkörper und eine auf die äußere Oberfläche des Trägerkörpers aufgebrachte katalytisch aktive Mischoxidmasse, welche

a) ein oder mehrere Oxide aus der Gruppe Titandioxid, Zirkoniumdioxid, Zinndioxid, Aluminiumoxid und

b) 0.1 bis 1.5 Gew-%, bezogen auf das Gewicht der Komponente a) und pro $m^2/g$ spezifischer Oberfläche der Komponente a), Vanadiumpentoxid enthält in einem Kreislaufverfahren wobei das Reaktionsausgangsgas in einem Reaktionsgaskreislauf zum Teil zurückgeführt wird und der Reaktionsgaskreislauf so ausgeführt wird, dass dem Reaktionsausgangsgas ein Teil der bei der Gasphasenoxidation entstandenen organischen Säuren entzogen wird, so dass der Säureanteil im zurückgeführten Anteil des Reaktionsausgangsgases aus 0,01 bis 12 vol.-% reduziert wird, dadurch gekennzeichnet, dass reines 2-Butanon und/oder 2-Butanon-haltige Rohprodukte aus Herstellungsverfahren für 2-Butanon als Kohlenstoffquelle eingesetzt wird.

Das erfindungsgemäße Verfahren liefert im Gegensatz zu den aus dem Stand der Technik bekannten Verfahren gleichermaßen hohe Säureausbeuten und somit gute Rohstoffausnutzung, hohe Rohsäurekonzentrationen, die sich in geringen Aufarbeitungskosten niederschlagen und gleichzeitig eine gesteigerte volumenbezogenen Säureproduktivitäten (hohe Raum/Zeit-Leistung), die sich wiederum in niedrigeren Anlagenkosten widerspiegeln.

Das erfindungsgemäße Verfahren eignet sich zur Herstellung von gesättigten Carbonsäuren mit ein bis vier C-Atomen, vorzugsweise zur Herstellung von Essigsäure und Ameisensäure, besonders bevorzugt von Essigsäure. Ein wesentlicher Vorteil der erfindungsgemäßen Vorgehensweise besteht darin, daß bei der Herstellung von Essigsäure, die dabei in geringen Mengen entstehenden Nebenprodukte als Wertstoffe, vor allem in Form von Ameisensäure, anfallen. Demgegenüber wird vor allem bei den aus dem Stand der Technik bekannten Verfahren mit Vollkontakt-Katalysatoren der intermediär entstehende Ameisensäureanteil zersetzt und es entstehen COx-Verbindungen, welche mittels Verbrennung entsorgt werden müssen.

2-Butanon ist eine gut verfügbare, großtechnisch hergestellte Chemikalie. Üblicherweise wird 2-Butanon durch Oxidation von Kohlenwasserstoffen in der flüssigen Phase, Hydratisierung von n-Butenen und Dehydrierung des resultierenden 2-Butanols zu 2-Butanon oder über eine Direktoxidation von n-Butenen mit Sauerstoff an Palladium-Katalysatoren in der flüssigen Phase zu 2-Butanon gewonnen. Letzeres Verfahren ist eng verwandt mit der Herstellung von Acetaldehyd aus Ethylen durch Direktoxidation mit Sauerstoff an Palladium-Katalysatoren in der flüssigen Phase.

Das erfindungsgemäße Verfahren eignet sich dabei gleichermaßen für den Einsatz von reinem 2-Butanon, 2-Butanon-haltigen Stoffgemischen und/oder 2-Butanon-haltigen Rohprodukten aus verschiedenen Herstellungsverfahren für 2-Butanon als Eduktstrom und somit als Kohlenstoffquelle für die Gaspahasenoxidation.

Da aufgrund der hohen Rohstoffkosten für Ethylen die Herstellung von Essigsäure aus Ethylen via Acetaldehyd unter ökonomischen Gesichtspunkten uninteressant geworden ist , bietet sich neben anderen beschriebenen Alternativprozessen zur Herstellung von Essigsäure, besonders der Übergang von der teuren Rohstoffbasis Ethylen auf die billige Rohstoffbasis der n-Butene an.

In einer bevorzugten Ausführungsform der Erfindung werden n-Butene mittels eines aus dem Stand der Technik bekannten Direktoxidationsprozess, wie er beispielsweise in DE 1049845 B, EP 0498 305, US 4,448,892 oder US 4,550,212 beschrieben wird, in 2-Butanon überführt. Die aus diesen Prozessen ohne weitere Aufarbeitung erhaltenen Produktgemische enthaltend 2-Butanon lassen sich direkt als Eduktströme im erfindungsgemäßen Verfahren einsetzen. Die weitere direkte Umsetzung dieser Primärproduktgemische aus der Herstellung von 2-Butanon zu Essigsäure ist angesichts der nicht notwendigen Aufarbeitung wirtschaftlich besonders interessant.

Im Eduktstrom neben 2-Butanon enthaltene Mengen an Wasser und aus der Direktoxidation von n-Butenen vorhandene nicht umgesetzte Edukte und Nebenprodukte sind für das erfindungsgemäße Verfahren unproblematisch.

Die Unanfälligkeit des erfindungsgemäßen Verfahren bezüglich eventueller Beimengungen anderer Stoffe oder Stoffgemische im 2 Butanon-Eduktstrom gestattet eine sehr kostengünstige Herstellung eines für das erfindungsgemäße Verfahren geeigneten 2-Butanon-haltigen Rohstoffgemisches nach einem aus dem Stand der Technik bekannten Verfahren der Direktoxidation an Palladium-Katalysatoren.

In einer besonders bevorzugten Ausführungsform der Erfindung werden die aus einem aus der Stand der Technik bekannten Verfahren zur Direktoxidation von 2- Butenen an Palladium-Katalysatoren primär als Rohprodukte anfallenden Wasser / 2-Butanon-Azeotrope als 2-Butanon-Eduktstrom eingesetzt. Eine aufwendige Entwässerung des primär anfallenden Wasser / 2-Butanon-Azeotrops ist nicht nötig und auch die Nebenprodukte der Reaktion, besonders der aufgrund des ähnlichen Siedepunktes schwer abzutrennende Butyraldehyd und Reste von unumgesetzten Edukten, müssen nicht aufwendig abgetrennt werden.

Bei der Umstellung von bestehenden Essigsäureanlagen nach dem Wacker/Hoechst-Verfahren von Ethen als Rohstoffbasis auf Butengemische als Rohstoffbasis kann ein Teil der bereits vorhandenen Anlage weiter genutzt werden.

Für die kostengünstige Herstellung von 2-Butanon werden bevorzugt preiswerte Rohstoffgemische aus der Petrochemie wie "$C_4$-Schnitt" (überwiegender Anteil an Butadien und i-Buten), "Raffinat 1" (überwiegender Anteil an i-Buten und n-Butenen) und "Raffinat 2" (überwiegender Anteil an Butanen, 1-Buten und 2-Butenen) als Ausgangsmaterial oder Mischungen die solche Kohlenwasserstoffe enthalten, gegebenenfalls nach einer Vorbehandlung z. B. einer Reinigung bzw. Hydrierung verwendet.

Die Reaktionstemperatur der Gasphasenoxidation beträgt im allgemeinen 100°C bis 400°C, vorzugsweise 150°C bis 300°C, besonders bevorzugt 180°C bis 250°C.

Die Reaktion wird im allgemeinen bei Drücken zwischen $1.2*10^5$ und $51*10^5$ Pa, bevorzugt zwischen $4*10^5$ und $41*10^5$ Pa, besonders bevorzugt zwischen $9*10^5$ und $17*10^5$ Pa durchgeführt.

Als Katalysatoren für das erfindungsgemäße Verfahren eignen sich alle Katalysatoren die allgemein für die partielle Oxidation von gesättigten und/oder ungesättigten $C_4$-Kohlenwasserstoffen zu Essigsäure beschrieben sind. Bevorzugt sind Mischoxidkatalysatoren die Vanadiumoxide enthalten. Besonders bevorzugt sind Schalenkatalysatoren bestehend aus einem inerten unporösen Trägerkörper und einer auf die äußere Oberfläche des Trägerkörpers aufgebrachten katalytisch aktiven Mischoxidmasse, welche

a) ein oder mehrere Oxide aus der Gruppe Titandioxid, Zirkoniumdioxid, Zinndioxid, Aluminiumoxid und

b) 0.1 bis 1.5 Gew-%, bezogen auf das Gewicht der Komponente a) und pro $m^2/g$ spezifischer Oberfläche der Komponente a), Vanadiumpentoxid enthält.

Zusätzlich kann die Komponente a) noch ein oder mehrere Oxide der Metalle aus der Gruppe Bor, Silicium, Hafnium, Niob, Wolfram, Lanthan und Cer enthalten. Bei der Dotierung der Komponente a) mit den genannten Oxiden sind diese im allgemeinen in einer Menge von 1 bis 30 Gew.-%, bezogen auf das Gesamtgewicht der Komponente a), enthalten.

In der Komponente b) kann ein Teil des Vanadiumpentoxids, vorzugsweise 10 bis 90 Gew.-%, durch ein oder mehrere Oxide von Molybdän, Chrom und Antimon ersetzt werden, und/oder als zusätzliche Komponente b) noch ein oder mehrere Oxide von Alkalimetallen, Erdalkalimetallen, Elementen der 5. und 6. Hauptgruppe des Periodensystems der Elemente (PSE) und der Übergangsmetalle enthalten sein. Im allgemeinen beträgt die Menge dieser Dotierstoffe 0.005 bis 15 Gew.-%, berechnet als Oxide und bezogen auf das Gesamtgewicht der Komponente b).

Bevorzugt werden Zusammensetzungen mit hoher Oberfläche der Komponente a) von 40 bis 300 $m^2/g$, wobei

gegebenenfalls noch Zinn- Niob- oder Wolframoxid enthalten sein können, und mit einer Komponente b), welche mit Mo, und/oder Cr, und/oder Sb und/oder Au dotiert ist.

Die katalytisch aktive Mischoxidmasse kann gegebenenfalls noch 10 bis 50 Gew.-%, bezogen auf das Gesamtgewicht der katalytisch aktiven Mischoxidmasse, inerte Verdünnungsmittel aus der Gruppe umfassend Siliciumdioxid, Siliciumcarbid und Graphit enthalten.

Die katalytisch aktive Mischoxidmasse ist vorzugsweise in einem Anteil von 1 bis 40 Gew.-%, vorzugsweise 5 bis 25 Gew.-%, jeweils bezogen auf das Gesamtgewicht aus Trägerkörper und aktiver Masse, als Schale auf die äußere Oberfläche des Trägerkörpers aufgebracht.

Die Schichtdicke beträgt vorzugsweise 10 bis 2000 μm, insbesondere 100 bis 1000 μm. Der Schalenkatalysator kann auch mehrere, sich in der Zusammensetzung unterscheidende, Schichten enthalten. Es können auch ein oder mehrere Bestandteile der Aktivkomponenten a) und b) in unterschiedlicher Konzentration in den einzelnen Schichten enthalten sein. In einer weiteren Ausführungsform enthält die innere Schicht nur die Komponente a) und die äußere Schicht die Komponenten a) und b). Eine denkbare Ausführungsform ist ein mehrschichtiger Schalenkatalysator, wobei die innere und die äußere Schicht jeweils die Komponenten a) und b) enthalten und für die innere Schicht eine höhere spezifische Oberfläche für die Komponente a) gewählt wird als für die äußere Schicht.

Geeignete Materialien für den inerten, unporösen Trägerkörper sind im allgemeinen alle sich unter den Betriebsbedingungen der Gasphasenoxidation inert verhaltenden und über den Betriebszeitraum stabilen unporösen Materialien. Beispiele hierfür sind Steatit, Duranit, Siliciumcarbid, Magnesiumoxid, Siliciumoxid, Silikate, Aluminate, Metalle wie Edelstahl, sowie gegebenenfalls Mischungen aus diesen Stoffen. Bevorzugt sind keramische Materialien, wie beispielsweise Steatit.

Die Formgestalt des inerten, unporösen Trägerkörpers des Schalenkatalysators ist beliebig. Beispiele für geeignete Formen sind Kugeln, Zylinder, Hohlzylinder, Quader, Tori, Sättel, Spindeln, Wendeln. Die Grundkörper können auch eine oder mehrere Ausnehmungen wie Mulden, Rillen, Löcher, oder auch abstehende Teile wie Zapfen, Spitzen, Stege besitzen. Weitere Beispiele sind Ringe, Ringsegmente, Steg-Ringe, durchbohrte Kugeln, Kugelsegmente. Ebenfalls als Träger geeignet sind geordnete Packungen wie Monolithe oder Kreuzkanalstrukturen. Bevorzugt sind Trägerformen mit möglichst hoher geometrischer Oberfläche pro Volumen, beispielsweise Ringe oder Hohlzylinder. In einer besonders bevorzugten Ausführungsform besitzen die inerten Trägerkörper die Form von Hohlzylindern, die eine oder mehrere Kerben an der oberen und/oder unteren Flachseite der Hohlzylinderwände besitzen, so daß der Innenraum der Hohlzylinder mit den die Hohlzylinder umgebenden Räumen über Öffnungen verbunden ist.

Die Abmessungen der Trägerkörper sind im allgemeinen durch die Reaktoren zur Gasphasenoxidation vorgegeben. Vorzugsweise haben die Formkörper eine Länge bzw. einen Durchmesser von 2 bis 20 mm. Die Wanddicke, beispielsweise im Fall von Ringen oder Hohlzylindern, beträgt zweckmäßigerweise 0,1 bis 4 mm.

Weitere Details zu bevorzugten Ausführungsformen der Katalysatoren sind in der EP 095135, der EP 0960874 und der EP 1108470 beschrieben, deren diesbezügliche Offenbarungen Teil dieser Anmeldung sein sollen und hiermit unter Bezugnahme eingeschlossen werden (incorporated by reference).

Als Reaktoren zur Durchführung des erfindungsgemäßen Verfahrens können im allgemeinen Ausführungen verwendet werden, die sich für die Durchführung von Oxidationsreaktionen in der Gasphase eignen und in der Lage sind die hohe Reaktionswärme ohne übermäßige Erwärmung des Reaktionsgemisches abzuführen. Das erfindungsgemäße Verfahren kann kontinuierlich oder intermitierend durchgeführt werden, das heißt die Zuführung des Reaktoreingangsgemisches kann mit konstantem Feed oder mit zyklisch variierender Feedzusammensetzung erfolgen. Das Gasgemisch kann vorzugsweise an dem Katalysator in einem Festbett, beispielsweise in einem Rohrbündelreaktor oder Hordenreaktor, oder in einem Fließ- bzw. Wirbelbett reagieren. Insbesondere bevorzugt sind gekühlte Rohrbündelreaktoren mit festem Katalysatorbett. Besonders bevorzugt sind Ausführungen mit zu Rohrbündel angeordneten Einzelrohren mit einem Rohrinnendurchmesser von 10 mm bis 50 mm und einer Rohrlänge von 1 m bis 6 m.

Die Strömungsgeschwindigkeit, bezogen auf das ungefüllte Rohr, in den Reaktionsrohren beträgt im allgemeinen zwischen 0,1 m/s und 10 m/s, bevorzugt zwischen 0,3 m/s und 5 m/s, besonders bevorzugt 0,5 bis 3 m/s.

Die Reaktionsrohre können mit Katalysator unterschiedlicher Zusammensetzung, Gestalt und Dimension gefüllt sein. Die Füllung kann vorzugsweise in axialer Richtung homogen oder zonenweise variabel in die Reaktionsrohre eingebracht sein. Jede Zone kann vorzugsweise einen statistisch verdünnten oder gemischten Katalysator enthalten.

Die für die Gasphasenoxidation notwendige Sauerstoffquelle ist im allgemeinen ein sauerstoffhaltiges Gas. Als sauerstoffhaltiges Gas wir vorzugsweise Luft, bevorzugt mit Sauerstoff angereicherte Luft und besonders bevorzugt reiner Sauerstoff verwendet werden. Bei dem erfingungsgemäßen Verfahren kann aber auch zusätzlich ein Inertgas, bevorzugt Stickstoff und/oder Argon anwesend sein.

Der Sauerstoffgehalt des dem Reaktor zugeführten Gasstroms beträgt vorzugsweise 1 bis 35 Vol.-%, besonders bevorzugt 3 bis 20 Vol.-%, insbesondere 4 bis 12 Vol.-%. Gegebenenfalls kann ein Inertgasanteil von 0 bis 25 Vol.-% zugeführt werden.

Der Volumenanteil von Wasserdampf des dem Reaktor zugeführten Reaktoreingangsgases beträgt im allgemeinen 5 bis 80 Vol.-%, vorzugsweise 5 bis 40 Vol.-%, besonders bevorzugt 5 bis 30 Vol.-% Wasserdampf.

Der Anteil an 2-Butanon im Reaktionsgas, gemessen am Reaktoreingang, beträgt vorzugsweise 0.3 bis 10 Vol.-%, besonders bevorzugt 0.5 bis 3.0 Vol.-%.

In einer bevorzugten Ausführungsform der Erfindung wird das erfindungsgemäße Verfahren mit Gasrückführung als Kreislaufverfahren betrieben. Bei Verfahren mit Gasrückführung hängt der Anteil an Kohlenoxiden und weiteren Reaktionsnebenprodukten im Reaktoreingangsgas von der Reaktionsführung und Säureabtrennung ab und beträgt im allgemeinen von 0 bis 90 Vol.-%, bevorzugt 10 bis 90 Vol.-%, besonders bevorzugt 50 bis 80 Vol.-%.

Die Anteile in Vol.-% der einzelnen Bestandteile des Reaktoreingangsgases addieren sich dabei jeweils auf 100 Vol.-%.

Als Vorrichtung zur Durchführung der erfindungsgemäßen Umsetzung können im allgemeinen Vorrichtungen mit einfachem Gasdurchgang durch den Reaktor und Kreislaufverfahren verwendet werden. Bei den Kreislaufverfahren sind Vorrichtungen bevorzugt bei denen aus dem rückgeführten Gasstrom bevorzugt Hochsieder, wie die Carbonsäuren Essig- und Ameisensäure gegenüber den Niedrigsiedern wie unumgesetzten Ketonen und niedrigsiedenden Zwischen- und Nebenprodukten abgeschieden werden. Die Rohsäure aus dem Reaktionsausgangsgas wird dabei bevorzugt mit einer Gegenstromwäsche, Gleichstromwäsche, Kreutzstromwäsche, Quenchkühlung, Partialkondensation oder einer Kombination dieser Verfahren abgetrennt. Weitere Details zu bevorzugten Ausführungsformen sind in der EP 0960875 und der EP 1035101 beschrieben, deren diesbezügliche Offenbarungen Teil dieser Anmeldung sein sollen und hiermit unter Bezugnahme eingeschlossen (incorporated by reference) werden sollen.

In einer besonders vorteilhaften Ausführungsform der Erfindung wird der Reaktionsgaskreislauf so ausgeführt, daß dem Reaktionsausgangsgas, also entweder dem den Reaktor verlassenden oder dem zurückgeführten Gasgemisch, ein Teil der bei der Gasphasenoxidation entstandenen organischen Säuren, primär Essigsäure und Ameisensäure, über einen Partialkondensator oder eine Gegenstromwäsche mit einem geeigneten Lösungsmittel, bevorzugt Wasser, ein Anteil an Säuren und anderen Verbindungen entzogen wird. Die Abtrennung wird dabei so ausgeführt, daß der Partialdruck dieser Säuren am Reaktoreingang niedrig bleibt, nicht umgesetztes 2-Butanon und weiter umsetzbare Zwischenprodukte wie Acetaldehyd, Aceton, Methylacetat, 2-Butanol etc. aber größtenteils im Kreisgas verbleiben und zum Reaktoreingang zurückgeführt werden.

Man kann dabei so vorgehen, daß bei einem Teil des Reaktorausgangsgases, im allgemeinen 60 bis 99,8 Gew.-%, vorzugsweise 90 bis 99,5 Gew.-%, der Säureanteil bis zu dem obengenannten Restsäuregehalt abgetrennt wird, und anschließend dieser Teil des Reaktorausgangsgases wieder in den Reaktor zurückgeführt wird. Der unbehandelte Teil des Reaktorausgangsgases wird verworfen und kann beispielsweise abgefackelt werden. Der Anteil an unbehandeltem Reaktorausgangsgas hängt davon ab, wieviel Kohlenoxide COx gebildet worden sind, weil diese über diesen Zweigstrom abgeführt werden müssen. Sie können danach mittels Verbrennung entsorgt werden.

Es kann auch so vorgegangen werden, daß dem Reaktorausgangsgas unmittelbar nach dem Austritt aus dem Reaktor der Säureanteil bis zu dem obengenannten Restanteil abgetrennt wird, und das so behandelte Reaktorausgangsgas ganz oder teilweise, vorzugsweise zu einem Anteil von 60 bis 99,8 Gew.-%, besonders bevorzugt von 90 bis 99,5 Gew.-% in den Reaktor zurückgeführt wird. Diese Ausführungsform ist besonders bevorzugt, da dabei die Zielprodukte, die Carbonsäuren, vorher weitgehend abgetrennt werden und nicht in die Verbrennung gehen.

Der rückgeführte Gasmassenstrom beträgt dabei im allgemeinen zwischen dem 1-fachen und dem 100-fachen des frisch zugeführten Eduktmassenstroms, bevorzugt zwischen dem 10-fachen und 80-fachen, besonders bevorzugt zwischen dem 30 bis 60-fachen.

Der Wasserdampfgehalt des Gasstroms, das den Absorber verläßt, wird im allgemeinen über die am Absorberausgang herrschende Temperatur und den Betriebsdruck festgelegt. Die Temperatur wird in der Regel über die aus dem Absorber abgeführte Wärmemenge und die Menge und Temperatur des Waschwasserstromes festgelegt und beträgt im allgemeinen 50°C bis 200°C. Der verbleibende Säuregehalt im Gasstrom der den Absorber verläßt, wird im allgemeinen über Druck und Temperatur, die Trennstufenzahl des Absorbers und die zugeführte Absorptionsmittelmenge (Wassereinspeisung) festgelegt. Im allgemeinem wird das Verfahren so ausgeführt, daß durch die Gegenstromwäsche die Restsäurekonzentration, des wieder in den Reaktor zurückgeführten Gasstroms auf 0,01 bis 12 Vol.-%, bevorzugt 0,1 bis 8 Vol.-% reduziert wird.

Das bei der Konzentrierung und Reinigung der Rohsäure anfallende Wasser wird teilweise, gegebenenfalls nach einer chemisch und/oder physikalischen Behandlung, in die Gegenstromabsorption zurückgespeist, so daß beim gesamten Prozeß kaum Abwasser anfällt.

Die abgetrennte Rohsäure wird im allgemeinen mit geeigneten üblichen Verfahren alleine oder in Kombination wie Flüssig-Flüssig-Extraktion, Extraktivrektifikation, Azeotroprektifikation, Rektifikation, Kristallisation und Membrantrennverfahren entwässert und gereinigt. Die vor einer weiteren Auftrennung der Rohsäure in ihre Reinsubstanzen abgetrennten Leichtsieder können ebenfalls, alleine oder zusammen mit Leichtsiedern aus der Reinigung und Konzentrierung ganz oder teilweise in den Gasphasenreaktor zurückgeführt werden.

Besonders geeignet für die Aufarbeitung der verdünnten Rohsäure sind bezüglich der Kosten optimierte Verfahren, wie sie in der DE 199 34 411 C1, der DE 199 34 410 C1 und der deutschen Patentanmeldung DE 10065466 beschrieben sind, deren diesbezügliche Offenbarungen Teil dieser Anmeldung sein sollen, werden hiermit unter Bezugnahme eingeschlossen (incorporated by reference).

Ein bevorzugtes Verfahren zur Trennung und Reinigung der verdünnten Rohsäure, eines wäßrigen Gemisches aus den Hauptkomponenten Essigsäure, Ameisensäure und Schwersiedern, ist die Extraktion, mittels eines Lösungsmittels, bevorzugt eine oder mehrere Verbindungen aus der Gruppe Ether, Ester, Ketone und Alkohole, besonders bevorzugt eine oder mehrer Verbindungen aus der Gruppe umfassend Methyltertiärbutylether, Diisopropylether, Ethylbutylether, Ethylacetat und Isopropylacetat in einem Kreisverfahren, dadurch gekennzeichnet, daß der Raffinatstrom mit einem Großteil des Wassers einer Lösungsmittelstripperkolonne zur Auskreisung des Wassers zugeführt wird und der Extraktstrom in eine Lösungsmitteldestillationskolonne geleitet wird, aus der in einem ersten Schritt über Kopf eine Mischung (A), bestehend aus Wasser und Lösungsmittel, über den Sumpf eine Mischung (B) bestehend aus Essigsäure, Ameisensäure und Hochsiedern abgetrennt wird, die Mischung (B) nach Abtrennung der Ameisensäure in einer gegebenenfalls mit Seitenabzug ausgestatteten Kolonne anschließend in einer Essigsäuredestillationskolonne in reine Essigsäure und Hochsieder aufgetrennt wird, und die Mischung (A) einem Phasentrenner zugeführt wird, wobei die wäßrige Phase mit Restanteilen an Lösungsmittel zur Lösungsmittelstripperkolonne, und die organische Phase zum Extraktor zurückgeführt wird. Ein weiteres bevorzugtes Verfahren ist die Extraktion, mittels eines Lösungsmittels, bevorzugt eine oder mehrere Verbindungen aus der Gruppe Ether, Ester, Ketone und Alkohole, besonders bevorzugt eine oder mehrer Verbindungen aus der Gruppe umfassend Methyltertiärbutylether, Diisopropylether, Ethylbutylether, Ethylacetat und Isopropylacetat in einem Kreisverfahren, dadurch gekennzeichnet, daß der Raffinatstrom mit einem Großteil des Wassers einer Lösungsmittelstripperkolonne zur Auskreisung des Wassers zugeführt wird und der Extraktstom in eine Lösungsmitteldestillationskolonne geleitet wird, aus der in einem ersten Schritt über Kopf eine Mischung (A) mit einem Großteil des Lösungsmittels, aus einem Seitenabzug eine Mischung (B) bestehend aus Ameisensäure, Wasser und Lösungsmittel und über den Sumpf eine Mischung (C) bestehend aus Essigsäure und Hochsiedern abgetrennt werden, und zur weiteren Verarbeitung die Mischung (B) in eine Ameisensäuredestillationskolonne übergeführt wird, und die Mischung (C) in eine Essigsäuredestillationskolonne übergeführt wird, anschließend in der Essigsäuredestillationskolonne über Kopf die reine Essigsäure isoliert wird, in der Ameisensäuredestillationskolonne am Sumpf die reine Ameisensäure isoliert wird und über Kopf ein Gemisch aus Lösungsmittel und Wasser abgezogen wird, welches zusammen mit der Mischung (A) nach Abtrennung des Wasseranteils, in den Extraktor zurückgeführt wird.

Eine weiteres bevorzugtes Verfahren ist die Extraktion, mittels eines Lösungsmittels, bevorzugt eine oder mehrere Verbindungen aus der Gruppe Ether, Ester, Ketone, Kohlenwasserstoffe und Alkohole, besonders bevorzugt eine oder mehrer Verbindungen aus der Gruppe umfassend Methyltertiärbutylether, Diisopropylether, Di-n-propylether, Ethylbutylether, Ethylacetat und Isopropylacetat in einem Kreisverfahren, wobei der Raffinatstrom mit einem Großteil des Wassers einer Lösungsmittelstripperkolonne zur Auskreisung des Wassers zugeführt wird und der Extraktstrom in eine Lösungsmitteldestillationskolonne geleitet wird, aus der in einem ersten Schritt über Kopf eine Mischung (A), bestehend aus Wasser und Lösungsmittel, über den Sumpf eine Mischung (B) bestehend aus Essigsäure, Ameisensäure und Hochsiedern abgetrennt wird, die Mischung (B) nach Abtrennung der Ameisensäure in einer Kolonne unter Einsatz eines Hilfsstoffes in der Art einer Azeotropdestillation anschließend in einer Essigsäuredestillationskolonne in reine Essigsäure und Hochsieder aufgetrennt wird, und die Mischung (A) einem Phasentrenner zugeführt wird, wobei die entstehende wässrige Phase mit Restanteilen an Lösungsmittel der Lösungsmittelstripperkolonne und die organische Phase dem Extraktor zurückgeführt wird.

Bei sehr hohen Essigsäurekonzentrationen in der Rohsäure sind für die Entwässerung apparativ unkompliziertere Verfahren wie die-Azeotroprektifikation günstiger.

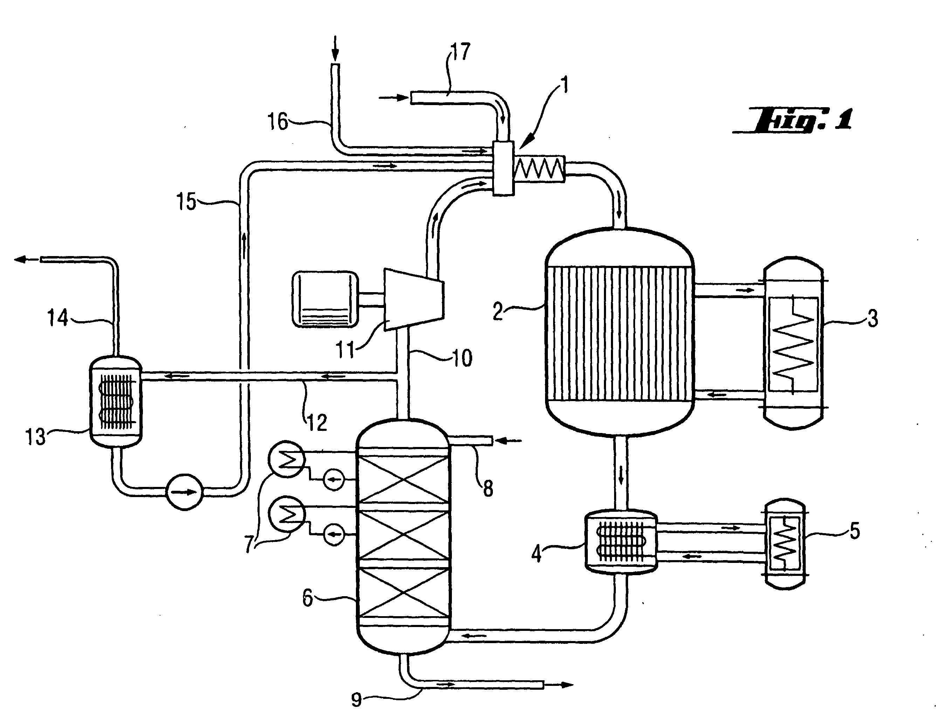

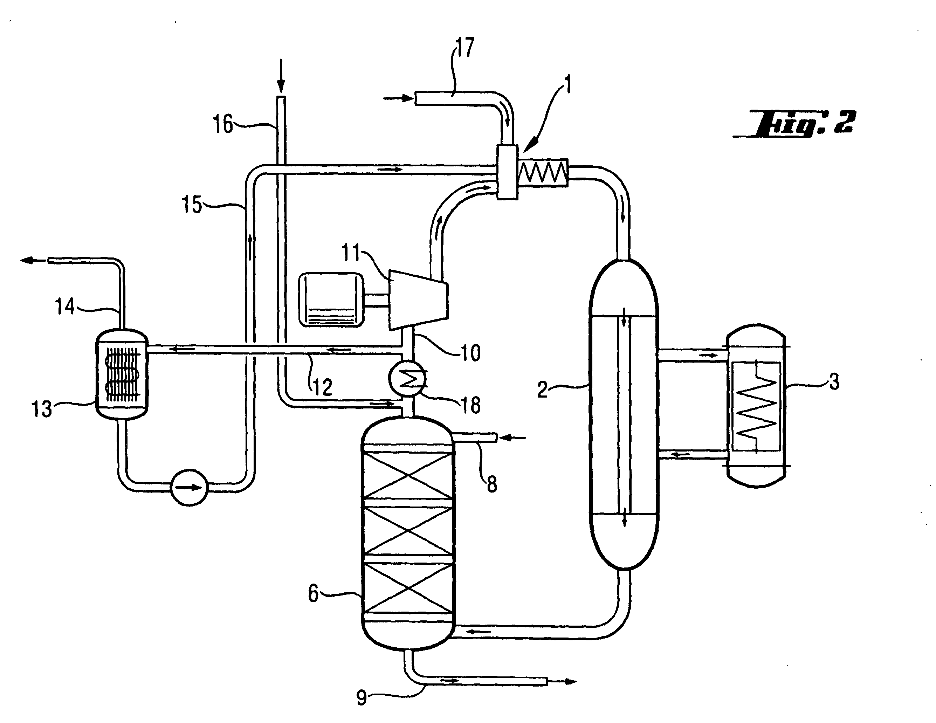

Figur 1 und 2 beschreiben in schematischer Darstellung Vorrichtungen zur Herstellung von gesättigten Carbonsäuren mit ein bis vier C-Atomen, insbesondere von Essigsäure durch Gasphasenoxidation von 2-Butanon an einem Schalenkatalysator nach dem erfindungsgemäßen Verfahren unter ganz oder teilweiser Rückführung des Reaktionsausgangsgases . Die Rohsäure wird dabei abgetrennt, bevorzugt durch eine Gegenstromabsorption mittels eines Lösungsmittels. Bevorzugtes Lösungsmittel ist dabei Wasser.

Hierbei wird über eine Mischzone (1) Sauerstoff (Leitung 17) und 2-Butanon mit dem rückgeführten Gasstrom vermischt und mit diesem zusammen dem Rohrbündelreaktor (2) zugeführt, der mittels einer Umlaufkühlung (3) gekühlt wird. Das den Reaktor verlassende Gasgemisch wird im Hauptstrom durch einen Gaskühler (4) geleitet der durch einen Umlaufkühler (5) gekühlt wird. Im Anschluß daran wird das Reaktionsgas in eine Absorptionskolonne (6) geleitet, die mit einem oder mehreren Kolonnenkühlern (7) ausgerüstet ist. Im obersten Kolonnenboden wird ein Lösungsmittel, bevorzugt Wasser, durch eine Rohrleitung (8) zugeleitet. In dieser Absorptionskolonne wird die Rohsäure durch Gegenstromwäsche abgetrennt und über ein Rohr (9) der weiteren Aufarbeitung zugeführt. Das restliche Reaktionsgas wird über ein Rohr (10) mittels einem Kreisgaskompressor (11) zur Mischzone zurückgeführt. Die Apparatur und das Verfahren können auch so ausgeführt werden, daß das kohlenstoffhaltige Edukt (2-Butanon) über Leitung (16) nicht auf die Mischdüse, sondern vor einer Kühleinrichtung (18) eingespeist wird (Fig. 2). Über Leitung (12) wird zur Aufrechterhaltung stationärer Bedingungen im Reaktionskreislauf ein geringer Abgasstrom entnommen und im Abgaskühler (13) abgekühlt wobei das anfallende Kondensat über Leitung (15) zum Reaktoreintritt zurückgeführt wird. Der über Leitung (14) entnommene Abgasstrom enthält überwiegend Kohlenoxide und Reste brennbare Kohlenwasserstoffe und kann einer thermischen Nutzung, beispielsweise einer Abgasverbrennung, einer stofflichen Nutzung oder einer sonstigen Abgasbehandlung zugeführt werden.

Die folgenden Beispiele dienen zur weiteren Erläuterungen der Erfindung.

Die Selektivität [in mol-%] wurde wie folgt berechnet:

$$\text{Essigsäureselektivität bezüglich 2-Butanon-Umsatz (mol-\%)}$$

$$= (((\text{mol/h Essigsäure in der Rohsäure})/2)/(\text{mol/h umgesetztes}$$

$$\text{2-Butanon})*100$$

$$\text{Ameisensäureselektivität bezüglich 2-Butanon-Umsatz (mol-\%)}$$

$$= (((\text{mol/h Ameisensäure in der Rohsäure})/4)/(\text{mol/h}$$

$$\text{umgesetztes 2-Butanon})*100$$

In den Beispielen verwendete Katalysatoren:

Katalysator I: Die Herstellung des verwendeten Katalysators erfolgte analog DE-A 19649426 dadurch gekennzeichnet, daß die aktive Masse aus Oxiden von Titan, Vanadium, Molybdän und Antimon der empirischen Formel $Ti_aV_bMo_cSb_dO_e$ besteht (a: 256; b:27; c: 4; d: 13; e: 611) und in einem Anteil von 14,4 Gew.-% plus 1,6 Gew.-% Graphit bezogen auf das Gewicht des Trägers auf Steatitringe der Dimension 7 mm Außendurchmesser * 4 mm Innendurchmesser * 4 mm Höhe aufgebracht ist.

Katalysator II: Die Herstellung des verwendeten Katalysators erfolgte analog DE-A 19649426 dadurch gekennzeichnet, daß die aktive Masse aus Oxiden von Titan, Vanadium, Molybdän und Antimon der empirischen Formel $Ti_aV_bMo_cSb_dO_e$ besteht (a: 91; b:7; c: 1; d: 3; e: 207) und in einem Anteil von 14,4 Gew.-% plus 1,6 Gew.-% Graphit bezogen auf das Gewicht des Trägers auf gekerbte Steatitringe analog der EP 1108470 der Dimension 7,3 mm Außendurchmesser * 4,1 mm Innendurchmesser * 4 mm Höhe mit je zwei Kerben mit 1,4 mm Breite und 2 mm Tiefe, auf Ober- und Unterseite, aufgebracht ist.

Die Versuche wurden in einer Apparatur entsprechend Figur 2 mit einem Ein-Rohr-Reaktor mit 19 mm Innendurchmesser des Reaktionsrohres und einer Absorptionskolonne mit strukturierter Packung, einem Innendurchmesser von 43 mm und einer Packungshöhe von 3240 mm mit thermostatisiertem Kopfteilkondensator als Kolonnenkühler durchgeführt.

Vergleichsbeispiel 1 (Oxidation von C4-Gemischen, niedriger n-Butengehalt):

In einen Reaktor mit einem Reaktionsrohrinnendurchmesser von 19 mm wurde ein Katalysator des Typs I mit einer Füllhöhe von 3200 mm eingebracht. Der Sauerstoffgehalt am Reaktoreintritt wurde automatisch auf 8 Vol.-% geregelt. Als Reaktionsfeed wurden 160 g/h C4-Gemisch mit 46 Gew.-% 1-Buten, 20 Gew.-% n-Butan, 13 Gew.-% t-2-Buten, 9 Gew.-% c-2-Buten, 4 Gew.-% i-Buten und 8 Gew.-% i-Butan eingespeist. Der Kreisgasfluß wurde so eingestellt, daß der Reaktor im stabilen Zustand einen Kreisgasfluß von 13000 g/h erreichte. Der Reaktor wurde bei $11*10^5$ Pa Druck und 195°C Kühlmitteltemperatur betrieben. Die Säureabtrennung aus dem Reaktionsgas erfolgte durch Absorption mit 1000 g/h Wasser (Aufgabe am Kopf) in einem Absorber mit strukturierter Packung, einem Innendurchmesser von 43 mm und einer Packungshöhe von 3240 mm bei einer Kopftemperatur des Absorbers von 130°C. Unter diesen Bedingungen wurde ein Butenumsatz von 97 % und ein Butanumsatz von 47 % erreicht. Die Essigsäureselektivität bezüglich des gesamt $C_4$-Umsatzes betrug 53 mol-%, die Ameisensäureselektivität bezüglich des gesamt $C_4$-Umsatzes betrug 10 mol-%. Die volumenspezifische Essigsäureproduktivität betrug 180 g/lh. Die Rohsäure enthielt 86 Gew.-% Wasser.

Vergleichsbeispiel 2 (Oxidation von C4-Gemischen, hoher n-Butengehalt):

In einen Reaktor mit einem Reaktionsrohrinnendurchmesser von 19 mm wurde ein Katalysator des Typs I mit einer Füllhöhe von 3000 mm eingebracht. Der Sauerstoffgehalt am Reaktoreintritt wurde automatisch auf 8 Vol.-% geregelt. Als Reaktionsfeed wurden 140 g/h C4-Gemisch mit 71 Gew.-% 1-Buten, 11 Gew.-% n-Butan, 8 Gew.-% t-2-Buten, 5 Gew.-% c-2-Buten, 2 Gew.-% i-Buten und 3 Gew.-% i-Butan eingespeist. Der Kreisgasfluß wurde so eingestellt, daß der Reaktor im stabilen Zustand einen Kreisgasfluß von 13000 g/h erreichte. Der Reaktor wurde bei $11*10^5$ Pa Druck und 190°C Kühlmitteltemperatur betrieben. Die Säureabtrennung aus dem Reaktionsgas erfolgte durch Absorption mit 640 g/h Wasser (Aufgabe am Kopf) in einem Absorber mit strukturierter Packung, einem Innendurchmesser von 43 mm und einer Packungshöhe von 3240 mm bei einer Kopftemperatur des Absorbers von 130°C. Unter diesen Bedingungen wurde ein Butenumsatz von 96 % und ein Butanumsatz von 66 % erreicht. Die Essigsäureselektivität bezüglich des gesamt $C_4$-Umsatzes betrug 60 mol-%, die Ameisensäureselektivität bezüglich des gesamt $C_4$-Umsatzes betrug 13 mol-%. Die volumenspezifische Essigsäureproduktivität betrug 190 g/lh. Die Rohsäure enthielt 76 Gew.-% Wasser.

Beispiel 1 (Oxidation von 2-Butanon(entspricht Methylethylketon)):

In einen Reaktor mit einem Reaktionsrohrinnendurchmesser von 19 mm wurde ein Katalysator des Typs I mit einer Füllhöhe von 3200 mm eingebracht. Der Sauerstoffgehalt am Reaktoreintritt wurde automatisch auf 8 Vol.-% geregelt. Als Reaktionsfeed wurden 340 g/h 2-Butanon eingespeist. Der Kreisgasfluß wurde so eingestellt, daß der Reaktor im stabilen Zustand einen Kreisgasfluß von 18000 g/h erreichte. Der Reaktor wurde bei $11*10^5$ Pa Druck und 185°C Kühlmitteltemperatur betrieben. Die Säureabtrennung aus dem Reaktionsgas erfolgte durch Absorption mit 1500 g/h Wasser (Aufgabe am Kopf) in einem Absorber mit strukturierter Packung, einem Innendurchmesser von 43 mm und einer Packungshöhe von 3240 mm bei einer Kopftemperatur des Absorbers von 130°C.
Unter diesen Bedingungen wurde ein 2-Butanonumsatz von 99,9 % erreicht. Die Essigsäureselektivität bezüglich des 2-Butanon-Umsatzes betrug 89 mol-%, die Ameisensäureselektivität bezüglich des 2-Butanon-Umsatzes betrug 5 mol-%. Die volumenspezifische Essigsäureproduktivität betrug 550 g/lh. Die Rohsäure enthielt 77 Gew.-% Wasser.

Beispiel 2 (Oxidation von 2-Butanon(entspricht Methylethylketon)):

In einen Reaktor mit einem Reaktionsrohrinnendurchmesser von 19 mm wurde ein Katalysator des Typs II mit einer Füllhöhe von 3000 mm eingebracht. Der Sauerstoffgehalt am Reaktoreintritt wurde automatisch auf 8 Vol.-% geregelt. Als Reaktionsfeed wurden 230 g/h 2-Butanon eingespeist. Der Kreisgasfluß wurde so eingestellt, daß der Reaktor im stabilen Zustand einen Kreisgasfluß von 13000 g/h erreichte. Der Reaktor wurde bei $11*10^5$ Pa Druck und 188°C Kühlmitteltemperatur betrieben.
Die Säureabtrennung aus dem Reaktionsgas erfolgte durch Absorption mit 410 g/h Wasser (Aufgabe am Kopf) in einem Absorber mit strukturierter Packung, einem Innendurchmesser von 43 mm und einer Packungshöhe von 3240 mm bei einer Kopftemperatur des Absorbers von 130°C.
Unter diesen Bedingungen wurde ein 2-Butanonumsatz von 99,8 % erreicht. Die Essigsäureselektivität bezüglich des 2-Butanon-Umsatzes betrug 83 mol-%, die Ameisensäureselektivität bezüglich des 2-Butanon-Umsatzes betrug 5 mol-%. Die volumenspezifische Essigsäureproduktivität betrug 370 g/lh. Die Rohsäure enthielt 52 Gew.-% Wasser.

Beispiele 3-9 (Oxidation von 2-Butanon (entspricht Methylethylketon):

Die Beispiele 3-9 wurden analog dem Beispiel 2 unter den in Tabelle 1 genannten Bedingungen mit den in der Tabelle genannten Ergebnissen durchgeführt.

Beispiel 10 (Oxidation von 2-Butanon(entspricht Methylethylketon)):

In einen Reaktor mit einem Reaktionsrohrinnendurchmesser von 19 mm wurde ein Katalysator des Typs II mit einer Füllhöhe von 1200 mm eingebracht. Der Sauerstoffgehalt am Reaktoreintritt wurde automatisch auf 8 Vol.-% geregelt. Als Reaktionsfeed wurden 250 g/h 2-Butanon eingespeist. Der Kreisgasfluß wurde so eingestellt, daß der Reaktor im stabilen Zustand einen Kreisgasfluß von 11000 g/h erreichte. Der Reaktor wurde bei $11*10^5$ Pa Druck und 173°C Kühlmitteltemperatur betrieben.
Die Säureabtrennung aus dem Reaktionsgas erfolgte durch Absorption mit 1500 g/h Wasser (Aufgabe am Kopf) in einem Absorber mit strukturierter Packung, einem Innendurchmesser von 43 mm und einer Packungshöhe von 3240 mm bei einer Kopftemperatur des Absorbers von 130°C.
Unter diesen Bedingungen wurde ein 2-Butanonumsatz von 99,5 % erreicht. Die Essigsäureselektivität bezüglich des 2-Butanon-Umsatzes betrug 83 mol-%, die Ameisensäureselektivität bezüglich des 2-Butanon-Umsatzes betrug 4 mol-%. Die volumenspezifische Essigsäureproduktivität betrug 1025 g/lh. Die Rohsäure enthielt 79 Gew.-% Wasser.

Beispiel 11 (Oxidation von 2-Butanon(entspricht Methylethylketon)):

In einen Reaktor mit einem Reaktionsrohrinnendurchmesser von 19 mm wurde ein Katalysator des Typs II mit einer Füllhöhe von 1200 mm eingebracht. Der Sauerstoffgehalt am Reaktoreintritt wurde automatisch auf 8 Vol.-% geregelt. Als Reaktionsfeed wurden 240 g/h 2-Butanon eingespeist. Der Kreisgasfluß wurde so eingestellt, daß der Reaktor im stabilen Zustand einen Kreisgasfluß von 11000 g/h erreichte. Der Reaktor wurde bei $11*10^5$ Pa Druck und 185°C Kühlmitteltemperatur betrieben.

Die Säureabtrennung aus dem Reaktionsgas erfolgte durch Absorption mit 30 g/h Wasser (Aufgabe am Kopf) in einem Absorber mit strukturierter Packung, einem Innendurchmesser von 43 mm und einer Packungshöhe von 3240 mm bei einer Kopftemperatur des Absorbers von 95°C.

Unter diesen Bedingungen wurde ein 2-Butanonumsatz von 97,5 % erreicht. Die Essigsäureselektivität bezüglich des 2-Butanon-Umsatzes betrug 78 mol-%, die Ameisensäureselektivität bezüglich des 2-Butanon-Umsatzes betrug 2 mol-%. Die volumenspezifische Essigsäureproduktivität betrug 890 g/lh. Die Rohsäure enthielt 16 Gew.-% Wasser.

## Tabelle 1

| BeispielNr. | Katalysator | Durchmesser Füllhöhe [mm] | Druck x10⁶ [Pa] | Kühltemperatur [°C] | Kreisgas fluß [kg/h] | C-Feed [g/h] | Absorber wasser [g/h] | Absorber kopf [°C] | Umsatz [%] | Selektivität [mol-%] | | Produktivi tät [g/lh] | Rohsäure [Gew.-%] |
|---|---|---|---|---|---|---|---|---|---|---|---|---|---|
| | | | | | | *R2 | | | *Buten/Butan | AcOH | HCOOH | AcOH | Wasser |
| VB 1 | I | 19*3200 | 11 | 195 | 13 | 160 *1 | 1000 | 130 | 97/47* | 53 | 10 | 180 | 86 |
| VB 2 | I | 19*3000 | ·11 | 190 | 13 | 140 *2 | 640 | 130 | 96/66* | 60 | 13 | 190 | 76 |
| 1 | I | 19*3200 | 11 | 185 | 18 | 340 | 1500 | 130 | 99,9 | 89 | 5 | 550 | 77 |
| 2 | II | 19*3000 | 11 | 188 | 13 | 230 | 410 | 130 | 99,8 | 83 | 5 | 370 | 52 |
| 3 | II | 19*3000 | 11 | 194 | 13 | 180 | 415 | 130 | 99,9 | 85 | 5 | 300 | 60 |
| 4 | II | 19*3000 | 11 | 189 | 14 | 220 | 205 | 130 | 99,6 | 82 | 4 | 350 | 36 |
| 5 | II | 19*3000 | 11 | 188 | 13 | 245 | 105 | 130 | 99,3 | 82 | 4 | 390 | 25 |
| 6 | II | 19*3000 | 11 | 189 | 11 | 245 | 50 | 109 | 99,7 | 84 | 3 | 400 | 17 |
| 7 | II | 19*3000 | 11 | 191 | 11 | 245 | 40 | 106 | 99,5 | 82 | 3 | 390 | 14 |
| 8 | II | 19*3000 | 11 | 182 | 11 | 245 | 0 | 90 | 98,7 | 82 | 3 | 390 | 9 |
| 9 | II | 19*3000 | 9 | 183 | 10 | 250 | 0 | 89 | 98,4 | 79 | 2 | 380 | 8 |
| 10 | II | 19*1200 | 11 | 173 | 11 | 250 | 1500 | 130 | 99,5 | 83 | 4 | 1025 | 79 |
| 11 | II | 19*1200 | 11 | 185 | 11 | 240 | 30 | 95 | 97,5 | 78 | 2 | 890 | 16 |

C4-1: 46 Gew.-% 1-Buten, 20 Gew.-% n-Butan, 13 Gew.-% t-2-Buten, 9 Gew.-% c-2-Buten, 4 Gew.-% i-Buten, 8 Gew.-% i-Butan

C4-2: 71 Gew.-% 1-Buten, 11 Gew.-% n-Butan, 8 Gew.-% t-2-Buten, 5 Gew.-% c-2-Buten, 2 Gew.-% i-Buten, 3 Gew.-% i-Butan

**Patentansprüche**

1. Verfahren zur Herstellung von gesättigten Carbonsäuren mit ein bis vier Kohlenstoffatomen durch Gasphasenoxidation an einem Schalenkatalysator enthaltend einen inerten unporösen Trägerkörper und eine auf die äußere Oberfläche des Trägerkörpers aufgebrachte katalytisch aktive Mischoxidmasse, welche

   a) ein oder mehrere Oxide aus der Gruppe Titandioxid, Zirkoniumdioxid, Zinndioxid, Aluminiumoxid und

   b) 0.1 bis 1.5 Gew-%, bezogen auf das Gewicht der Komponente a) und pro $m^2$/g spezifischer Oberfläche der Komponente a), Vanadiumpentoxid enthält

   in einem Kreislaufverfahren wobei das Reaktionsausgangsgas in einem Reaktionsgaskreislauf zum Teil zurückgeführt wird und der Reaktionsgaskreislauf so ausgeführt wird, dass dem Reaktionsausgangsgas ein Teil der bei der Gasphasenoxidation entstandenen organischen Säuren entzogen wird, so dass der Säureanteil im zurückgeführten Anteil des Reaktionsausgangsgases auf 0,01 bis 12 Vol.-% reduziert wird, **dadurch gekennzeichnet, dass** reines 2-Butanon und/oder 2-Butanon-haltige Rohprodukte aus Herstellungsverfahren für 2-Butanon als Kohlenstoffquelle eingesetzt wird.

2. Verfahren nach Anspruch 1, **dadurch gekennzeichnet, dass** die katalytisch aktive Mischoxidmasse des Schalenkatalysators als Komponente a) zusätzlich noch ein oder mehrere Oxide der Metalle aus der Gruppe Bor, Silicium , Hafnium, Niob, Wolfram, Lanthan und Cer, in einer Menge von 1 bis 30 Gew-%, bezogen auf das Gesamtgewicht der Komponente a) enthält.

3. Verfahren nach Anspruch 1 bis 2, **dadurch gekennzeichnet, dass** in der katalytisch aktiven Mischoxidmasse des Schalenkatalysators als Komponente b) ein Teil des Vanadiumpentoxids durch ein oder mehrere Oxide von Molybdän, Chrom und Antimon ersetzt ist, und/oder als zusätzliche Komponente b) noch ein oder mehrere Oxide von Alkalimetallen, Erdalkalimetallen, Elementen der 5. und 6. Hauptgruppe des Periodensystems der Elemente (PSE) und der Übergangsmetalle enthalten sind.

4. Verfahren nach Anspruch 1 bis 3, **dadurch gekennzeichnet, dass** der Schalenkatalysator ein oder mehrere Schichten der katalytisch aktiven Mischoxidmasse enthält.

5. Verfahren nach Anspruch 1 bis 4, **dadurch gekennzeichnet, dass** der Schalenkatalysator mehrere Schichten enthält, wobei die innere Schicht nur Komponente a) enthält und die äußere Schicht die Komponenten a) und b).

6. Verfahren nach Anspruch 1 bis 5, **dadurch gekennzeichnet, dass** der Schalenkatalysator mehrere Schichten enthält, wobei die innere und äußere Schicht jeweils die Komponenten a) und b) enthalten, und für die innere Schicht eine höhere spezifische Oberfläche für die Komponente a) gewählt wird, als für die äußere Schicht.

7. Verfahren nach Anspruch 1 bis 6, **dadurch gekennzeichnet, dass** die inerten Trägerkörper des Schalenkatalysators die Form von Hohlzylindern aufweisen, die eine oder mehrere Kerben an der oberen und/oder unteren Flachseite der Hohlzylinderwände besitzen, so daß der Innenraum der Hohlzylinder mit den die Hohlzylinder umgebenden Räumen über Öffnungen verbunden ist.

8. Verfahren nach Anspruch 1 bis 7, **dadurch gekennzeichnet, dass** ein Gasgemisch, enthaltend 1 bis 35 Vol.-% Sauerstoff, 0.3 bis 10 Vol.-% 2-Butanon allein oder im Gemisch mit anderen organischen Verbindungen, 5 bis 80 Vol.-% Wasserdampf und 0 bis 90 Vol.-% Kohlenoxide, so dass sich die Anteile der einzelnen Bestandteile in Vol.-% des Gasgemisches auf jeweils 100 % addieren, bei einer Temperatur von 100°C bis 400°C, und einem Überdruck von $1.2*10^5$ bis $51*10^5$ Pa an dem Schalenkatalysator umgesetzt wird.

9. Verfahren nach Anspruch 1 bis 8, **dadurch gekennzeichnet, dass** bei 60 bis 99.8 Gew-% des Reaktorausgangsgases der Säureanteil bis zu dem genannten Restsäuregehalt abgetrennt wird und anschließend dieser Teil des Reaktorausgangsgases wieder in den Reaktor zurückgeführt wird.

10. Verfahren nach Anspruch 1 bis 9, **dadurch gekennzeichnet, dass** dem Reaktorausgangsgas unmittelbar nach dem Austritt aus dem Reaktor der Säureanteil bis zu dem genannten Restanteil abgetrennt wird, und das so behandelte Reaktorausgangsgas ganz oder teilweise in den Reaktor zurückgeführt wird.

11. Verfahren nach Anspruch 1 bis 10, **dadurch gekennzeichnet, dass** der Anteil des zurückgeführten Gasmassenstroms zwischen dem 1-fachen und dem 100-fachen des frisch zugeführten Eduktmassenstroms beträgt.

12. Verfahren nach Anspruch 1 bis 11, wobei das Reaktionsausgangsgas in einem Reaktionsgaskreislauf zum Teil zurückgeführt wird und dabei durch einen Abtrennschritt die Säurekonzentration im zurückgeführten Anteil verringert wird, **dadurch gekennzeichnet, dass** die Rohsäure aus dem Reaktionsausgangsgas durch eine Gegenstromwäsche, Gleichstromwäsche, Kreutzstromwäsche, Quenchkühler, einen Partialkondensator oder eine Kombination dieser Verfahren abgetrennt wird.

13. Verfahren nach Anspruch 1 bis 12, **dadurch gekennzeichnet dass** die Trennung und Reinigung des erhaltenen wässrigen Gemisches aus den Hauptkomponenten Essigsäure, Ameisensäure und Schwersiedern durch Extraktion in einem Extraktor, mittels eines Lösungsmittels in einem Kreisverfahren erfolgt, wobei der Raffinatstrom aus dem Extraktor mit einem Großteil des Wassers einer Lösungsmittelstripperkolonne zur Auskreisung des Wassers zugeführt wird und der Extraktstrom in eine Lösungsmitteldestillationskolonne geleitet wird, aus der in einem ersten Schritt über Kopf eine Mischung (A) mit einem Großteil des Lösungsmittels, aus einem Seitenabzug eine Mischung (B) bestehend aus Ameisensäure, Wasser und Lösungsmittel und über den Sumpf eine Mischung (C) bestehend aus Essigsäure und Schwersiedern abgetrennt werden, und zur weiteren Verarbeitung die Mischung (B) in eine Ameisensäuredestillationskolonne übergeführt wird, und die Mischung (C) in eine Essigsäuredestillationskolonne übergeführt wird, anschließend in der Essigsäuredestillationskolonne über Kopf die reine Essigsäure isoliert wird, in der Ameisensäuredestillationskolonne am Sumpf die reine Ameisensäure isoliert wird und über Kopf ein Gemisch aus Lösungsmittel und Wasser abgezogen wird, welches zusammen mit der Mischung (A) nach Abtrennung des Wasseranteils, in den Extraktor zurückgeführt wird.

14. Verfahren nach Anspruch 1 bis 13, **dadurch gekennzeichnet dass** die Trennung und Reinigung des erhaltenen wäßrigen Gemisches aus den Hauptkomponenten Essigsäure, Ameisensäure und Schwersiedern durch Extraktion in einem Extraktor, mittels eines Lösungsmittels in einem Kreisverfahren erfolgt, wobei der Raffinatstrom aus dem Extraktor mit einem Großteil des Wassers einer Lösungsmittelstripperkolonne zur Auskreisung des Wassers zugeführt wird und der Extraktstrom in eine Lösungsmitteldestillationskolonne geleitet wird, aus der in einem ersten Schritt über Kopf eine Mischung (A), bestehend aus Wasser und Lösungsmittel, über den Sumpf eine Mischung (B) bestehend aus Essigsäure, Ameisensäure und Schwersiedern abgetrennt wird, die Mischung (B) nach Abtrennung der Ameisensäure in einer gegebenenfalls mit Seitenabzug ausgestatteten Kolonne anschließend in einer Essigsäuredestillationskolonne in reine Essigsäure und Hochsieder aufgetrennt wird, und die Mischung (A) einem Phasentrenner zugeführt wird, wobei die wäßrige Phase mit Restanteilen an Lösungsmittel zur Lösungsmittelstripperkolonne, und die organische Phase zum Extraktor zurückgeführt wird.

15. Verfahren nach Anspruch 1 bis 14, **dadurch gekennzeichnet dass** die Trennung und Reinigung des erhaltenen wäßrigen Gemisches aus den Hauptkomponenten Essigsäure, Ameisensäure und Schwersiedern durch Extraktion in einem Extraktor, mittels eines Lösungsmittels in einem Kreisverfahren erfolgt, wobei der Raffinatstrom aus dem Extraktor mit einem Großteil des Wassers einer Lösungsmittelstripperkolonne zur Auskreisung des Wassers zugeführt wird und der Extraktstrom in eine Lösungsmitteldestillationskolonne geleitet wird, aus der in einem ersten Schritt über Kopf eine Mischung (A), bestehend aus Wasser und Lösungsmittel, über den Sumpf eine Mischung (B) bestehend aus Essigsäure, Ameisensäure und Hochsiedern abgetrennt wird, die Mischung (B) nach Abtrennung der Ameisensäure in einer Kolonne unter Einsatz eines Hilfsstoffes in der Art einer Azeotropdestillation anschließend in einer Essigsäuredestillationskolonne in reine Essigsäure und Hochsieder aufgetrennt wird, und die Mischung (A) einem Phasentrenner zugeführt wird, wobei die entstehende wässrige Phase mit Restanteilen an Lösungsmittel der Lösungsmittelstripperkolonne, und die organische Phase dem Extraktor zurückgeführt wird.

16. Verfahren nach den Ansprüchen 1 bis 15, **dadurch gekennzeichnet, dass** die Entwässerung des erhaltenen wässrigen Gemisches aus den Hauptkomponenten Essigsäure, Ameisensäure und Schwersiedern durch eine Azeotroprektifikation erfolgt.

17. Verfahren nach den Ansprüchen 1 bis 16, **dadurch gekennzeichnet, dass** als Edukte die bei der Herstellung von 2-Butanon anfallenden 2-Butanon-haltigen Primärproduktgemische direkt als Eduktströme eingesetzt werden.

18. Verfahren nach den Ansprüchen 1 bis 17, **dadurch gekennzeichnet, dass** als Edukte die aus der Direktoxidation von 2-Butenen an Palladium-Katalysatoren als Primärproduktgemische anfallenden Wasser/2-Butanon-Azeotrope direkt als Eduktströme eingesetzt werden.

**Claims**

1. Process for preparing saturated carboxylic acids having from one to four carbon atoms by gas-phase oxidation over a coated catalyst comprising an inert nonporous support body and a catalytically active mixed oxide composition comprising

   a) one or more oxides selected from the group consisting of titanium dioxide, zirconium dioxide, tin dioxide and aluminium oxide, and

   b) from 0.1 to 1.5% by weight, based on the weight of the component a) and per $m^2/g$ of specific surface area of the component a), of vanadium pentoxide,

   applied to the external surface of the support body, in a circulation process wherein the reactor outlet gas is partly recirculated in a reaction gas circuit which is configured so that part of the organic acids formed in the gas-phase oxidation are separated off from the reactor outlet gas so that the proportion of acids in the part of the reactor outlet gas which is recirculated is reduced to from 0.01 to 12% by volume, **characterized in that** pure 2-butanone and/or 2-butanone-containing crude products from production processes for 2-butanone are used as carbon source.

2. Process according to Claim 1, **characterized in that** the catalytically active mixed oxide composition of the coated catalyst further comprises, as part of component a), one or more oxides of metals selected from the group consisting of boron, silicon, hafnium, niobium, tungsten, lanthanum and cerium, in an amount of from 1 to 30% by weight, based on the total weight of the component a).

3. Process according to Claim 1 or 2, **characterized in that**, in the catalytically active mixed oxide composition of the coated catalyst, part of the vanadium pentoxide of component b) is replaced by one or more oxides of molybdenum, chromium and antimony, and/or one or more oxides of alkali metals, alkaline earth metals, elements of main groups 5 and 6 of the Periodic Table of the Elements and the transition metals are present as additional component b).

4. Process according to any of Claims 1 to 3, **characterized in that** one or more layers of the catalytically active mixed oxide composition are present in the coated catalyst.

5. Process according to any of Claims 1 to 4, **characterized in that** the coated catalyst has a plurality of layers of which the inner layer comprises only component a) and the outer layer comprises the components a) and b).

6. Process according to any of Claims 1 to 5, **characterized in that** the coated catalyst has a plurality of layers of which the inner layer and the outer layer each comprise the components a) and b) and a higher specific surface area of the component a) is selected for the inner layer than for the outer layer.

7. Process according to any of Claims 1 to 6, **characterized in that** the inert support bodies of the coated catalyst have the shape of hollow cylinders which have one or more notches on the upper and/or lower end face of the hollow cylinder walls, so that the interior space of the hollow cylinders is joined to the spaces surrounding the hollow cylinders via openings.

8. Process according to any of Claims 1 to 7, **characterized in that** a gas mixture comprising from 1 to 35% by volume of oxygen, from 0.3 to 10% by volume of 2-butanone either alone or in admixture with other organic compounds, from 5 to 80% by volume of water vapour and from 0 to 90% by volume of carbon oxides, so that the percentages by volume of the individual constituents of the gas mixture in each case add up to 100%, is reacted over the coated catalyst at a temperature of from 100°C to 400°C and a superatmospheric pressure of from $1.2 \times 10^5$ to $51 \times 10^5$ Pa.

9. Process according to any of Claims 1 to 8, **characterized in that** the acids are separated off down to the specified residual acid content from 60-99.8% by weight of the reactor outlet gas and this part of the reactor outlet gas is subsequently recirculated to the reactor.

10. Process according to any of Claims 1 to 9, **characterized in that** the acids are separated off down to the specified residual content from the reactor outlet gas immediately after it leaves the reactor and the reactor outlet gas which has been treated in this way is wholly or partly recirculated to the reactor.

**11.** Process according to any of Claims 1 to 10, **characterized in that** the mass flow of the recirculated gas stream is from 1 to 100 times the mass flow of the fresh feed introduced.

**12.** Process according to any of Claims 1 to 11, wherein the reactor outlet gas is partly recirculated in a reaction gas circuit and the acid concentration in the recirculated part is reduced by means of a separation step, **characterized in that** the crude acid is separated off from the reactor outlet gas by means of a countercurrent scrub, a cocurrent scrub, a cross-stream scrub, quench cooling, partial condensation or a combination of these methods.

**13.** Process according to any of Claims 1 to 12, **characterized in that** the separation and purification of the aqueous mixture of the main components acetic acid, formic acid and high boilers obtained is carried out by extraction in an extractor by means of a solvent in a circulation process, where the raffinate stream from the extractor containing the major part of the water is fed to a solvent stripping column to remove the water and the extract stream is passed to a solvent distillation column from which, in a first step, a mixture (A) containing the major part of the solvent is separated off at the top, a mixture (B) comprising formic acid, water and solvent is taken off at a side offtake and a mixture (C) comprising acetic acid and high boilers is separated off at the bottom, and the mixture (B) is passed to a formic acid distillation column for further work-up and the mixture (C) is passed to an acetic acid distillation column, the pure acetic acid is subsequently isolated via the top of the acetic acid distillation column, the pure formic acid is isolated at the bottom of the formic acid distillation column and a mixture of solvent and water is taken off at the top of the formic acid distillation column and is, together with the mixture (A) after the water has been separated off, recirculated to the extractor.

**14.** Process according to any of Claims 1 to 13, **characterized in that** the separation and purification of the aqueous mixture of the main components acetic acid, formic acid and high boilers obtained is carried out by extraction in an extractor by means of a solvent in a circulation process, where the raffinate stream from the extractor containing the major part of the water is fed to a solvent stripping column to remove the water and the extract stream is passed to a solvent distillation column from which, in a first step, a mixture (A) comprising water and solvent is separated off at the top and a mixture (B) comprising acetic acid, formic acid and high boilers is separated off at the bottom, the mixture (B) is, after the formic acid has been separated off in a column which may be equipped with a side offtake, subsequently separated into pure acetic acid and high boilers in an acetic acid distillation column, and the mixture (A) is passed to a phase separator and the aqueous phase containing residual solvent is returned to the solvent stripping column and the organic phase is recirculated to the extractor.

**15.** Process according to any of Claims 1 to 14, **characterized in that** the separation and purification of the aqueous mixture of the main components acetic acid, formic acid and high boilers obtained is carried out by extraction in an extractcr by means of a solvent in a circulation process, where the raffinate stream from the extractor containing the major part of the water is fed to a solvent stripping column to remove the water and the extract stream is passed to a solvent distillation column from which, in a first step, a mixture (A) comprising water and solvent is separated off at the top and a mixture (B) comprising acetic acid, formic acid and high boilers is separated off at the bottom, the mixture (B) is, after the formic acid has been separated off in a column using an auxiliary in the manner of an azeotropic distillation, subsequently separated into pure acetic acid and high boilers in an acetic acid distillation column, and the mixture (A) is passed to a phase separator and the resulting aqueous phase containing residual solvent is returned to the solvent stripping column and the organic phase is recirculated to the extractor.

**16.** Process according to any of Claims 1 to 15, **characterized in that** the dewatering of the aqueous mixture of the main components acetic acid, formic acid and high boilers obtained is carried out by means of an azeotropic rectification.

**17.** Process according to any of Claims 1 to 16, **characterized in that** the 2-butanone-containing primary product mixtures obtained in the preparation of 2-butanone are used directly as feedstocks.

**18.** Process according to any of Claims 1 to 17, **characterized in that** the water/2-butanone azeotropes obtained as primary product mixtures in the direct oxidation of 2-butenes over palladium catalysts are used directly as feedstocks.

**Revendications**

**1.** Procédé en vue de la préparation d'acides carboxyliques saturés, ayant de un à quatre atomes de carbone, par oxydation en phase gazeuse sur un catalyseur à coquille contenant un corps de support inerte non poreux et une masse d'oxydes mixtes, active du point de vue catalytique, appliquée à la surface externe du corps de support, qui contient

a) un ou plusieurs oxydes du groupe du dioxyde de titane, du dioxyde de zirconium, du dioxyde d'étain, de l'oxyde d'aluminium et
b) de 0,1 à 1,5% en poids, par rapport au poids du composant a) et par $m^2/g$ de surface spécifique du composant a),de pentoxyde de vanadium,

dans un procédé en circuit fermé, le gaz de sortie de réaction étant reconduit en partie dans un circuit fermé de gaz de réaction et le circuit fermé de gaz de réaction étant réalisé de telle sorte que l'on soustrait au gaz de sortie de réaction une partie des acides organiques produits lors de l'oxydation en phase gazeuse, de façon à ce que la proportion d'acide dans la proportion reconduite du gaz de sortie de réaction soit réduite à de 0,01 à 12% en volume, **caractérisé en ce que** l'on utilise, en tant que source de carbone, du 2-butanone pur et/ou des matières premières contenant du 2-butanone provenant de procédés de fabrication pour le 2-butanone.

2. Procédé selon la revendication 1, **caractérisé en ce que** la masse d'oxydes mixtes, active du point de vue catalytique, du catalyseur à coquille contient, en tant que composant a), en outre encore un ou plusieurs oxydes des métaux venant du groupe du bore, du silicium, du hafnium, du niobium, du tungstène, du lanthane et du cérium, dans une quantité allant de 1 à 30% en poids, par rapport au poids total du composant a).

3. Procédé selon les revendications 1 à 2, **caractérisé en ce que**, dans la masse d'oxydes mixtes, active du point de vue catalytique, du catalyseur à coquille, une partie du pentoxyde de vanadium, en tant que composant b), est remplacée par un ou plusieurs oxydes du molybdène, du chrome et de l'antimoine, et/ou qu'encore un ou plusieurs oxydes des métaux alcalins, des métaux alcalino-terreux, des éléments des 5ème et 6ème groupes principaux du système périodique des éléments (SPE) et des métaux de transition sont contenus en tant que composant supplémentaire b).

4. Procédé selon les revendications 1 à 3, **caractérisé en ce que** le catalyseur à coquille contient une ou plusieurs couches de la masse d'oxydes mixtes, active du point de vue catalytique.

5. Procédé selon les revendications 1 à 4, **caractérisé en ce que** le catalyseur à coquille contient plusieurs couches, la couche interne ne contenant que le composant a) et la couche externe contenant les composants a) et b).

6. Procédé selon les revendications 1 à 5, **caractérisé en ce que** le catalyseur à coquille contient plusieurs couches, la couche interne et externe contenant, à chaque fois, les composants a) et b) et **en ce que** l'on choisit, pour la couche interne, une superficie spécifique plus élevée pour le composant que celle choisie pour la couche externe.

7. Procédé selon les revendications 1 à 6, **caractérisé en ce que** les corps de support inertes du catalyseur à coquille présentent la forme de cylindres creux, qui possèdent une ou plusieurs entailles sur la face plate supérieure et/ou inférieure des parois de cylindres creux, de telle sorte que l'espace interne des cylindres creux soit raccordé par des ouvertures aux espaces entourant les cylindres creux.

8. Procédé selon les revendications 1 à 7, **caractérisé en ce que** l'on fait réagir, sur le catalyseur à coquille, à une température de 100°C à 400°C, et à une surpression de $1,2*10^5$ à $51*10^5$ Pa, un mélange gazeux, contenant de 1 à 35% en volume d'oxygène, de 0,3 à 10% en volume de 2-butanone seulement ou en mélange à d'autres composés organiques, de 5 à 80% en volume de vapeur d'eau et de 0 à 90% en volume d'oxydes de carbone, de telle sorte que les proportions des constituants individuels en % en volume du mélange gazeux s'additionnent, à chaque fois, à 100%.

9. Procédé selon les revendications 1 à 8, **caractérisé en ce que**, pour de 60 à 99,8% en poids du gaz de sortie de réaction, la proportion acide est séparée jusqu'à la teneur résiduelle en acide citée et que cette partie du gaz de sortie du réacteur est ensuite à nouveau reconduite au réacteur.

10. Procédé selon les revendications 1 à 9, **caractérisé en ce que** la proportion d'acide est séparée du gaz de sortie du réacteur, immédiatement après la sortie du réacteur, jusqu'à la proportion restante citée et **en ce que** le gaz de sortie du réacteur ainsi traité est complètement ou partiellement reconduit dans le réacteur.

**11.** Procédé selon les revendications 1 à 10, **caractérisé en ce que** la proportion du courant massique de gaz reconduite est comprise entre 1 fois et 100 fois le courant massique de produit de départ fraîchement introduit.

**12.** Procédé selon les revendications 1 à 11, le gaz de sortie de réaction étant reconduit en partie dans le circuit fermé de gaz de réaction et la concentration en acide dans la portion reconduite étant réduite par une étape de séparation, **caractérisé en ce que** l'acide brut provenant du gaz de sortie de réaction est séparé par un lavage à contre-courant, un lavage à co-courant, un lavage à courant transversal, un refroidisseur de trempe, un condenseur partiel ou une combinaison de ces procédés.

**13.** Procédé selon les revendications 1 à 12, **caractérisé en ce que** la séparation et le nettoyage du mélange aqueux obtenu à partir des composants principaux acide acétique, acide formique et fractions lourdes se fait dans un procédé en circuit fermé par extraction dans un extracteur à l'aide d'un solvant, le courant de raffinat étant reconduit hors de l'extracteur avec une grande partie de l'eau en direction d'une colonne de stripage de solvants en vue de l'élimination de l'eau et le courant d'extrait étant conduit dans une colonne de distillation des solvants, hors de laquelle, dans une première étape, par la tête un mélange (A) avec une grande partie du solvant, d'un prélèvement latéral un mélange (B), se composant d'acide formique, d'eau et de solvant, et par le puits un mélange (C), se composant d'acide acétique et de fractions lourdes, sont séparés, et en vue d'un traitement ultérieur, le mélange (B) étant acheminé dans une colonne de distillation de l'acide formique, et le mélange (C) étant conduit dans une colonne de distillation de l'acide acétique, l'acide acétique pur étant ensuite isolé dans la colonne de distillation de l'acide acétique par l'intermédiaire de la tête, l'acide formique pur étant isolé dans la colonne de distillation de l'acide formique au puits et par l'intermédiaire de la tête, un mélange de solvants et d'eau étant prélevé, lequel mélange est reconduit, conjointement au mélange (A) après séparation de la portion d'eau, dans l'extracteur.

**14.** Procédé selon les revendications 1 à 13, **caractérisé en ce que** la séparation et le nettoyage du mélange aqueux obtenu à partir des composants principaux acide acétique, acide formique et fractions lourdes se fait dans un procédé à circuit fermé, par extraction dans un extracteur à l'aide d'un solvant, le courant de raffinat provenant de l'extracteur étant conduit avec une grande partie de l'eau à une colonne de stripage des solvants en vue de l'élimination de l'eau et le courant d'extrait étant conduit dans une colonne de distillation des solvants, hors de laquelle, dans une première étape, par la tête un mélange (A), se composant d'eau et de solvant, par le puits un mélange (B), se composant d'acide acétique, d'acide formique et de fractions lourdes sont séparées, le mélange (B), après séparation de l'acide formique dans une colonne équipée, le cas échéant, d'un prélèvement latéral, étant ensuite séparée dans une colonne de distillation de l'acide acétique en acide acétique pur et en fractions lourdes, et le mélange (A) étant conduit à un séparateur de phases, la phase aqueuse avec les portions restantes de solvants étant conduite à la colonne de stripage des solvants, et la phase organique étant reconduite à l'extracteur.

**15.** Procédé selon les revendications 1 à 14, **caractérisé en ce que** la séparation et le nettoyage du mélange aqueux obtenu à partir des composants principaux acide acétique, acide formique et fractions lourdes se fait dans un procédé en circuit fermé, par extraction dans un extracteur à l'aide d'un solvant, le courant de raffinat provenant de l'extracteur étant conduit avec une grande partie de l'eau à une colonne de stripage des solvants en vue de l'élimination de l'eau et le courant d'extrait étant conduit dans une colonne de distillation des solvants, hors de laquelle, dans une première étape, par la tête, un mélange (A), se composant d'eau et de solvants, par le puits un mélange (B), se composant d'acide acétique, d'acide formique et de fractions lourdes, sont séparés, le mélange (B), après séparation de l'acide formique dans une colonne avec utilisation d'un adjuvant dans le genre d'un distillation azéotropique, étant ensuite séparé dans une colonne de distillation d'acide acétique en acide acétique pur et en fractions lourdes, et le mélange (A) étant conduit à un séparateur de phases, la phase aqueuse se formant avec des parties résiduelles de solvant étant reconduit à la colonne de stripage des solvants et la phase organique étant reconduite à l'extracteur.

**16.** Procédé selon les revendications 1 à 15, **caractérisé en ce que** l'extraction d'eau du mélange aqueux obtenu à partir des composants principaux acide acétique, acide formique et fractions lourdes se fait par l'intermédiaire d'une rectification azéotropique.

**17.** Procédé selon les revendications 1 à 16, **caractérisé en ce que** l'on utilise, en tant que produits de départ, les mélanges de produits primaires, contenant de la 2-butanone, se formant lors de la fabrication de 2-butanone, directement en tant que produits de départ.

**18.** Procédé selon les revendications 1 à 17, **caractérisé en ce que** l'on utilise, en tant que produits de départ, l'azéotrope eau/2-butanone se formant en tant que mélange de produits primaires à partir de l'oxydation directe du 2-butène sur des catalyseurs au palladium, directement en tant que courants de produits.

Fig. 1

Fig. 2

EP 1 318 136 B1